(19) Europäisches Patentamt
European Patent Office
Office européen des brevets

(11) **EP 2 496 288 B1**

(12) **EUROPÄISCHE PATENTSCHRIFT**

(45) Veröffentlichungstag und Bekanntmachung des Hinweises auf die Patenterteilung:
**30.04.2014 Patentblatt 2014/18**

(21) Anmeldenummer: **10779237.6**

(22) Anmeldetag: **03.11.2010**

(51) Int Cl.:
*A61M 5/162* (2006.01)    *A61M 5/38* (2006.01)
*A61M 5/40* (2006.01)

(86) Internationale Anmeldenummer:
**PCT/EP2010/006691**

(87) Internationale Veröffentlichungsnummer:
**WO 2011/054497 (12.05.2011 Gazette 2011/19)**

(54) **DOPPELLUMIGER EINSTECHDORN MIT GASSPERRELEMENT FÜR EIN HÄMODIALYSESCHLAUCHSET**

DOUBLE-LUMEN SPIKE CONNECTOR WITH A GAS-BLOCKING ELEMENT FOR A HEMODIALYSIS TUBE SET

CONNECTEUR À AIGUILLE DOUBLE-LUMIÈRE AVEC UN ÉLÉMENT BARRIÈRE A GAZ POUR TUYAU À HÉMODIALYSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.11.2009 DE 102009051945**

(43) Veröffentlichungstag der Anmeldung:
**12.09.2012 Patentblatt 2012/37**

(73) Patentinhaber: **Fresenius Medical Care Deutschland GmbH**
**61352 Bad Homburg (DE)**

(72) Erfinder:
• **FINI, Massimo**
**I-26020 Palazzo Pignano (IT)**
• **WEISS, Stefan**
**61352 Bad Homburg (DE)**
• **TERPIN, Andreas**
**60385 Frankfurt/Main (DE)**
• **VENERONI, Alain**
**I-26016 Spino d'Adda (CR) (IT)**

(74) Vertreter: **Weiß, Stefan**
**Fresenius Medical Care AG & Co. KGaA**
**Global Patents & IP**
**Siemensstrasse 21**
**61352 Bad Homburg (DE)**

(56) Entgegenhaltungen:
**WO-A1-96/29113        US-A1- 2003 040 700**
**US-A1- 2008 097 315    US-B1- 6 261 267**

**Beschreibung**

Die Erfindung betrifft ein System zur Administration von Medikamenten in extrakorporale Kreisläufe. Im Besonderen betrifft die Erfindung einen Adapter, der den direkten Anschluss einer handelsüblichen Durchstechflasche an einen extrakorporalen Kreislauf ermöglicht unter Vermeidung der Einbringung von Luft in den Kreislauf.

Kontinuierliche extrakorporale Kreisläufe werden in der Medizin meist zu einer Behandlung des Blutes außerhalb des Körpers des Patienten genutzt. Insbesondere bei der Hämodialyse wird dem Patienten Blut in einem kontinuierlichen Prozess entnommen, das entnommene Blut mittels eines Dialysators gereinigt und umgehend dem Blutkreislauf des Patienten rückgeführt. Das Blut des Patienten wird dabei mittels mindestens einer Pumpe befördert. Es zirkuliert üblicherweise in einem extrakorporalen Schlauchset, das Mittel zur arteriellen Entnahme, Abschnitte zur Blutförderung durch Einlage in die Blutpumpe, Anschlüsse für den Dialysator, eine venöse Tropfkammer, Mittel zur venösen Rückführung des Bluts an den Patienten sowie eine venöse Tropfkammer enthält und miteinander verbindet. Als Alternative zu einem solchen Schlauchset werden auch Kassettensysteme benutzt, bei denen zumindest Teile der Leitungswege, Anschlüsse und die Tropfkammer in einer spritz- oder blasgeformten Plastikkassette integriert werden. Die prinzipiellen Leitungswege bleiben aber auch in einem Kassettensystem erhalten.

Das Krankheitsbild von mittels extrakorporalen Kreisläufen behandelten Patienten erfordert meist die intravenöse Verabreichung von Medikamenten. Zur Behandlung von Dialysepatienten übliche Medikamente umfassen z.B. Vitamin D sowie rekombinates Erythropoietin und Eisenpräparate zur Behandlung der Anämie.

Solche Medikamente werden üblicherweise in Durchstechflaschen zur Verfügung gestellt. Eine Verabreichung erfolgt durch das Klinikpersonal indem die entsprechende Dosis des Medikaments in eine Einmalspitze überführt wird und über dafür im extrakorporalen Schlauchset oder Kassettenset vorgesehene Zuspritzstellen verabreicht wird. Auf diese Weise können die Medikamente zeitsparend während der extrakorporalen Behandlung und ohne die Notwendigkeit einer zusätzlichen Konnektion des Patienten zur Infusion administriert werden.

Die Zuspritzstellen sind dabei meist als T-Stücke konzipiert, die durch ein Septum oder einen absperrbaren Luerkonnektor zugänglich sind. Nachteilig an der Verabreichung durch solche Zugänge ist die Verwendung eines zusätzlichen Einmalteils, der Spritze, zur Überführung des Medikaments. Darüber hinaus birgt die Verabreichung über eine mit einer Kanüle ausgestatteten Spritze ein Verletzungsrisiko für das Klinikpersonal und einer damit in Verbindung stehenden Problematik bei der Entsorgung der Einmalspritzen. Die meisten Medikamente werden dabei als Bolus verabreicht. Es gibt aber auch Medikamente, wie z.B. das Eisenpräparat Venofer, die bevorzugt über einen längeren Zeitraum verabreicht werden. In einem solchen Fall ist die manuelle Injektion zeitaufwendig, weil das Klinikpersonal über den gesamten Zeitraum der verlängerten Injektion für diese Aufgabe zur Verfügung stehen muss.

Ein ähnliches Problem wie bei der Zugabe von Medikamenten stellt sich auch bei der Zugabe von antikoagulierenden Mitteln. Außerhalb des Körpers neigt das Blut zur Koagulation. Obwohl die Biokompatibilität von Schlauch- oder Kassettensets sowie von Dialysatoren ständig verbessert wird, kann die Koagulation des Blutes ohne den Zusatz antikoagulierender Mittel nicht verhindert werden. Die Zugabe von antikoaglulierenden Mitteln, wie Heparin oder Citrat, erfolgt bei der Dialysebehandlung ebenfalls über den extrakorporalen Kreislauf und bevorzugt nicht als Bolus, über einen gewissen Zeitraum. Zu diesem Zweck enthalten die meisten Dialysemaschinen eine Vorrichtung zur Infusion des antikoagulierenden Mittels, meist Heparin. Solche Heparinpumpen sind üblicherweise als Spritzenpumpen ausgebildet, die automatisch gesteuert werden.

DE 38 37 298 offenbart die Anordnung einer heparingefüllten Spritze im Unterdruckbereich des extrakorporalen Kreislaufs und ein passives Einsaugen des Heparins in den extrakorporalen Kreislauf.

US 5,330,425 und EP 966 631 offenbaren Schlauchsets mit einer blasgeformten venösen Tropfkammer mit verschiedenen Zuspritzstellen für Medikamente an der Tropfkammer und im Schlauchset hinter der Tropfkammer.

US 4,500,309 offenbart die Infusion von Citratlösung in den arteriellen Bereich des extrakorporalen Kreislaufs und die Infusion von Calciumlösung in den venösen Bereich des extrakorporalen Kreislaufs.

EP 532 432 offenbart die Infusion einer Substituatlösung in den extrakorporealen Kreislauf. WO 87/07159 offenbart ein doppellumiges Überleitstück zur Konnektion einer Durchstechflasche.

Aus der US 2008/0097315 A1 ist eine Durchstechvorrichtung mit Tropfkammer für Infusionsflaschen zur intravenösen Verabreichung von Medikamenten bekannt.

Es ist Aufgabe der Erfindung eine sichere Infusion in den extrakorporalen Kreislauf zu ermöglichen ohne die Notwendigkeit einer weiteren mechanischen Pumpe.

Es ist weiterhin eine Aufgabe der Erfindung die Infusion direkt aus einer handelsüblichen Durchstechflasche ohne die Notwendigkeit der Überführung in eine Spritze automatisiert durchzuführen.

Eine weitere Aufgabe ist es die Infusion des Medikaments in einem geringen Volumen durchzuführen.

Die Aufgaben werden durch eine Vorrichtung nach Anspruch 1 gelöst.

Eine medizinische Vorrichtung mit einer Durchstechvorrichtung zum Einführen in eine Durchstechflasche (22) zur Medikamentenaufbewahrung mit einem ersten Leitungsweg (10) und einem zweiten Leitungsweg (11),

wobei der erste Leitungsweg (10) so ausgelegt ist, dass er an einem Ende (17) in das innere Lumen der Durchstechflasche mündet, wenn die Durchstechvorrichtung vollständig in Durchstechflasche eindringt, und mit dem anderen Ende an die Leitungswege eines extrakorporalen Blutkreislaufs (19) einer Hämodialysemaschine anschließt,

und wobei der zweite Leitungsweg (11) so ausgelegt ist, dass er an einem Ende (18) in das innere Lumen der Durchstechflasche mündet, wenn die Durchstechvorrichtung vollständig in Durchstechflasche eindringt und mit dem anderen Ende mit einem Gasreservoir (14) so in Verbindung steht, dass eine Belüftung über den zweiten Leitungsweg (11) stattfinden kann, und wobei im ersten Leitungsweg (10) ein Gassperrelement (12, 23) so angebracht ist, dass Flüssigkeiten das Gassperrelement passieren können, das Gassperrelement für Gase aber unpassierbar ist.

Die Vorrichtung kann an verschiedenen Stellen des extrakorporalen Blutkreislaufs eingesetzt werden, um den Inhalt einer Medikamentendurchstechflasche direkt in den extrakorporalen Blutkreislauf einzubringen.

Figur 1 zeigt schematisch einen extrakorporalen Blutkreislauf einer Hämodialyse.

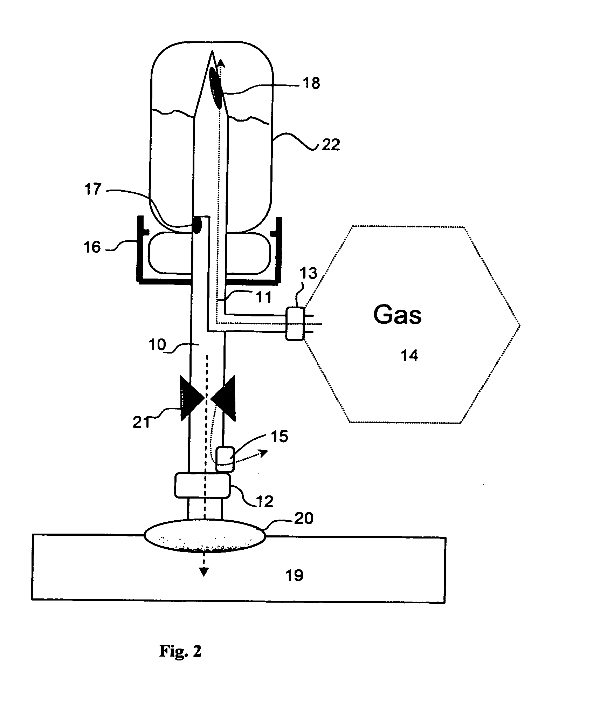

Figur 2 zeigt schematisch eine erfindungsgemäße Vorrichtung, ein Überleitstück mit einem doppellumigen Spike mit einer aufgesteckten Durchstechflasche und der Verbindung zum extrakorporalen Kreislauf. In dem ersten Leitungsweg ist eine hydrophile Membran angeordnet.

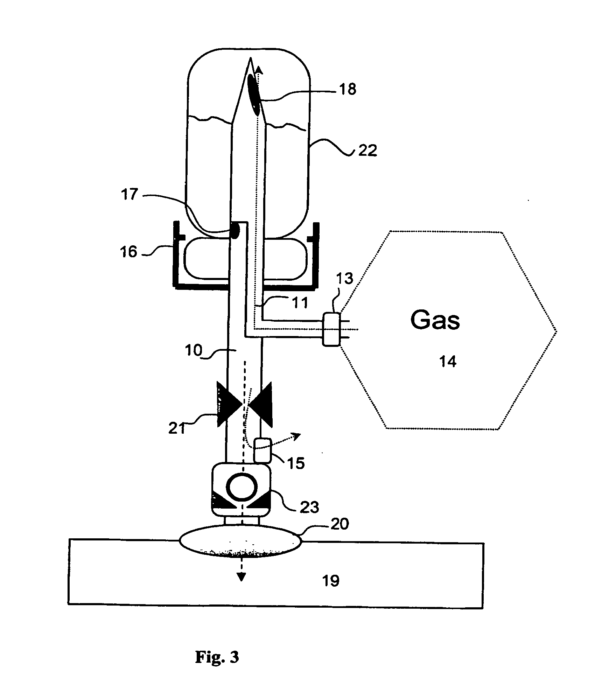

Figur 3 zeigt schematisch eine alternative erfindungsgemäße Vorrichtung, ein Überleitstück mit einem doppellumigen Spike mit einer aufgesteckten Durchstechflasche und der Verbindung zum extrakorporalen Kreislauf. In dem ersten Leitungsweg ist ein Schwimmerventil angeordnet.

In einem extrakorporalen Kreislauf zur Hämodialyse wird dem Patienten das Blut üblicherweise an einer arteriell-venösen Fistel entnommen. Der Teil des extrakorporalen Kreislaufs vor dem Dialysator (5) wird als arterieller Kreislauf (1) bezeichnet, der Teil des extrakorporalen Kreislaufs nach dem Dialysator wird als venöser Kreislauf (3, 4) bezeichnet. Eine Blutfördereinrichtung (2) ist normalerweise im arteriellen Teil des extrakorporalen Kreislaufs angeordnet. Die Blutfördereinrichtung ist häufig als eine Schlauchrollenpumpe ausgestaltet. Im venösen Teil des extrakorporalen Blutkreislaufs ist meist eine venöse Tropfkammer (6) angeordnet. Die venöse Tropfkammer dient zur Entlüftung des Bluts. In dem extrakorporalen Kreislauf, insbesondere aber am Dialysator können kleine Mengen Luft ins Blut geraten, die sich im Blut zu Luftbläschen zusammenfinden und nicht wieder reinfundiert werden dürfen, weil die Infusion von Luft in den natürlichen Blutkreislauf Schmerzen verursachen und im schlimmsten Fall zur Embolie führen kann. In der venösen Tropfkammer werden diese daher Luftbläschen eliminiert.

Die Blutfördereinrichtung fördert das Blut durch den extrakorporalen Kreislauf. Sie entwickelt vor dem Teil des Kreislaufs vor der Pumpe einen Sog, wogegen das Blut in dem Teil nach der Pumpe durch Druck vorwärts bewegt wird. Daher herrscht im dem arteriellen Kreislauf vor der Pumpe ein Unterdruck, wogegen im Teil des Kreislaufs nach der Pumpe ein Überdruck herrscht. Der Druck im venösen Kreislauf wird durch Messung an der venösen Tropfkammer ermittelt.

Der extrakorporale Blutkreislauf findet in den meisten Systemen vollständig innerhalb eines Schlauchsets oder eines Kassettensets statt. Ein solches Set beinhaltet die Anschlüsse zur Konnektion des Patienten und zur Konnektion mit dem Dialysator. Die Tropfkammer und Vorrichtungen für diagnostische Messungen sind ebenfalls im Set integriert.

Diese allgemeine Funktionsweise gilt auch für die Hämofiltration und die Hämodiafiltration, die eine höhere Ultrafiltrationsrate besitzen und eine Teil der aus dem Blut abfiltrierten Flüssigkeit durch Substituatlösung ersetzen. Im Rahmen der vorliegenden Erfindung werden daher Hämofiltration und Hämodiafiltration unter dem Oberbegriff Hämodialyse zusammengefasst.

In den Schlauchsets gibt es üblicherweise auch Zuspritzstellen für Medikamente. Dabei bieten sich folgende Zuspritzstellen an:

Eine Zuspritzstelle (7) im arteriellen Blutkreislauf (1) vor der Blutfördereinrichtung.
Eine Zuspritzstelle (8) im venösen Blutkreislauf (3) vor der venösen Tropfkammer.
Eine Zuspritzstelle (9) im venösen Blutkreislauf (4) nach der venösen Tropfkammer.

Die Zuspritzstelle, zumeist in Form eines T-Stücks mit Septum-Zugang, absperrbarem Luerzugang oder beiden Zugangsarten, im extrakorporalen Blutkreislauf vor der Blutfördereinrichtung wird allgemein als arterielle Zugabestelle (7) bezeichnet. Bei einem Blutfluss von Null herrscht an dieser Stelle ein leichter Überdruck von zirka 100 mbar. Bei einem behandlungsüblichen Blutfluss von zirka 250 bis 500 ml/min ergeben sich an dieser Stelle Unterdruckverhältnisse im Bereich von zirka 50 bis 300 mbar. Durch Unregelmäßigkeiten in der Pumpenfunktion der Blutfördereinrichtung ergeben sich deutliche Druckschwankungen. Der real gegebene Druck ist auch abhängig vom Druckabfall beim Durchströmen der Kanüle und der Schlauchleitung und auch von der geodätischen Höhendifferenz zwischen Patientenanschluss und Entnahmestelle abhängig.

Insgesamt eignet sich diese Stelle im extrakorporalen Blutkreislauf dazu, Infusionslösungen oder Medikamentenlösungen über Schwerkraft-Infusionseinrichtungen einsaugen zu lassen.

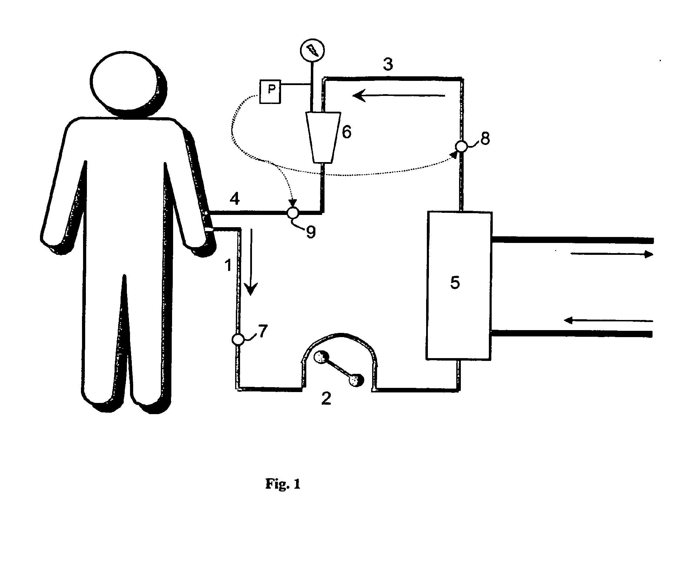

Erfindungsgemäß eignet sich zur Applikation eines Medikaments, das in einer handelsüblichen Durchstechflasche (22) zur Verfügung gestellt wird, eine medizinische Vorrichtung mit einer Durchstechvorrichtung mit zwei Leitungswegen. Die Durchstechvorrichtung mit zwei Leitungswegen kann in Form von 2 separaten Hohlnadeln, Kanülen oder Spikes, die in die medizinische Vorrichtung integriert sind, ausgeführt sein. Bevorzugt ist die Durchstechvorrichtung jedoch ein doppellumiger Spike. Alle im Folgenden beschriebenen Ausführungsformen, bei denen der Begriff Spike verwendet wird, beziehen sich sowohl auf die Durchstechvorrichtung mit 2 separaten Durchstechelementen als auch auf bevorzugte Ausführungsform des doppellumigen Spikes. Wie in Figur 1 dargestellt wird der Spike bei erfindungsgemäßer Nutzung vollständig in die Durchstechflasche (22) eingeführt. Üblicherweise wird die Durchstechflasche dabei mit ihrer Öffnung nach unten eingesetzt. Das Einführen des Spikes ist vollständig, wenn beide Leitungswege des Spikes in den Innenraum der Durchstechflasche münden.

In einer bevorzugten Ausführungsform ist an dem Spike eine Aufnahmevorrichtung (16) zur zumindest teilweisen Aufnahme der Durchstechflache vorgesehen. Die Aufnahmevorrichtung ist weiterhin vorteilhafterweise so ausgestaltet, dass sie die Durchstechflasche in ihrer Position hält. Zu diesem Zweck können an der Aufnahmevorrichtung Rastnasen vorgesehen sein, die hinter dem Hals der Durchstechflasche einrasten, um die Durchstechflasche gegen ein ungewolltes Abkoppeln abzusichern. Idealerweise ist die Aufnahmevorrichtung weiterhin so ausgestaltet, dass man eine Kappe dicht aufsetzen kann. Eine solche Kappe soll den Zugang vor und nach der Benutzung dicht abschließen, um die Verunreinigung durch die Umgebung so gering wie möglich zu halten. Zuspritzstellen in den extrakorporalen Kreislauf müssen einem hohen Sterilitätsstandard genügen.

Wenn der Spike vollständig in die Durchstechflasche (22) eindringt, müssen beide Leitungswege (10, 11) in das Innere der Durchstechflasche münden. Die Auflagefläche der Aufnahmevorrichtung gewährleistet dabei idealerweise, dass der Spike immer gleich tief in die Durchstechflasche eindringt.

In einer bevorzugten Ausführungsform ist die medizinische Vorrichtung so ausgestaltet, dass ein Ventil die Fluidverbindung des ersten Leitungswegs und/oder des zweiten Leitungswegs nur dann öffnet, wenn eine Durchstechflasche vollständig in die medizinische Vorrichtung eingeführt ist. Solche Aufnahmevorrichtungen zur sicheren Konnektion einer Durchstechflasche sind im Stand der Technik bekannt, beispielsweise offenbart die italienische Patentanmeldung TO2009A000455 eine geeignete Vorrichtung.

In einer besonders bevorzugten Ausführungsform ist die medizinische Vorrichtung so auf die Form der Durchstechflasche und auf die Dicke des die Durchstechflasche abdichtenden Septums eingestellt, dass der erste Leitungsweg (10) an einem Ende (17) so in die Durchstechflasche mündet, dass der Abstand zwischen dem Ende des Leitungswegs (17) und dem durchstochenen Septum möglichst gering ist, bevorzugt ist der Abstand zwischen dem Ende des ersten Leitungswegs und dem Septum geringer als 3 mm und besonders bevorzugt geringer als 1mm. Das Ende (18) des zweiten Leitungswegs (11) mündet in der bevorzugten Ausführungsform so in die Durchstechflasche, dass das Ende (18) ebenfalls in der Durchstechflasche liegt, wenn der Spike vollständig in die Durchstechflasche eindringt. In der bevorzugten Ausführungsform ist der Abstand des Endes (18) des zweiten Leitungswegs zum Septum größer als der Abstand des Endes (17) des ersten Leitungswegs, besonders bevorzugt ist der Abstand größer als 3 mm und besonders bevorzugt größer als 6 mm. In einer speziell bevorzugten Ausführungsform liegt das Ende (18) des zweiten Leitungswegs oberhalb des Flüssigkeitsspiegels der in der Durchstechflasche enthaltenen Flüssigkeit. In einer weiteren bevorzugten Ausführungsform ist der Durchmesser des ersten Leitungswegs an jeder Stelle größer als der Durchmesser des zweiten Leitungswegs.

Das andere Ende des ersten Leitungswegs (10) schließt an den extrakorporalen Blutkreislauf (19) an. Auf diese Weise wird eine Verbindung geschaffen zwischen der Durchstechflasche (22) und dem extrakorporalen Blutkreislauf (19), die eine direkte Applikation des Inhalts der Durchstechflasche in den extrakorporalen Kreislauf erlaubt.

Wenn der Inhalt der Durchstechflasche an der arteriellen Zugabestelle (7) in den extrakorporalen Kreislauf eingesaugt wird, muss das auf diese Weise entfernte Volumen ersetzt werden. Andernfalls entsteht im inneren der starren Durchstechflasche ein Unterdruck gegenüber den Druckverhältnissen im extrakorporalen Kreislauf. Wenn der Druck im Inneren der Durchstechflasche geringer ist als der im extrakorporalen Kreislauf, endet der Fluss des Inhalts der Durchstechflasche in den extrakorporalen Kreislauf. Um dies zu vermeiden ist der zweite Leitungsweg (11) an ein Gasreservoir (14) angeschlossen, aus dem das Volumen des in den extrakorporalen Kreislauf einfließenden Inhalts der Durchstechflasche durch Gas substituiert wird, um so für einen Druckausgleich zu sorgen. Im einfachsten Fall könnte die umgebende Luft ein Gasreservoir darstellen. Erfindungsgemäß wird das Gasreservoir durch einem starren oder einem flexiblen mit Gas befüllten Behälter dargestellt. In diesem Fall ist der Behälter bevorzugt flexibel. Als weitere Alternative kann erfindungsgemäß eine Gas führende Leitung das Gasreservoir bilden.

Falls es sich beim dem bereitgestellten Gas nicht um steriles Gas handelt, wie etwa im Falle der umgebenden Luft, ist im zweiten Leitungsweg idealerweise ein gasdurchlässiger Sterilfilter (13) vorgesehen. Solche Sterilfilter sind dem Fachmann bekannt und werden in der Medizintechnik häufig zur Belüftung von Leitungswegen unter Einhaltung der Sterilität eingesetzt. Sterilfilter zeichnen sich in erster Linie dadurch aus, dass sie eine Porengröße beziehungsweise eine Maschenweite besitzen, die es Mikroorganismen nicht erlaubt diese Barriere zu passieren. Bevorzugt ist eine Porengröße von weniger als 0,3 μm. In einer bevorzugten Ausführungsform handelt es sich bei dem Sterilfilter um einen Hydrophobfilter. Ein Hydrophobfilter mit einem hydrophoben Filtermaterial wird nicht durch wässrige Flüssigkeiten benetzt oder genässt und lässt keine solchen Flüssigkeiten passieren. Auf diese Weise wird vermieden, dass der Inhalt der Durchstechflasche durch den zweiten Leitungsweg austritt.

Das Gas des Gasreservoirs sollte im Idealfall nicht mit dem Inhalt der Durchstechflasche reagieren. So kann es sich beispielsweise empfehlen bei einem leicht oxidierbaren Inhalt statt Luft ein inertes Gas, wie zum Beispiel Stickstoff, aus dem Reservoir zur Verfügung zu stellen.

Das Gasreservoir sollte bevorzugt so bemessen sein, dass es das vollständige Entleeren der Durchstechflasche erlaubt, also im Falle von Normaldruck ein gleich großes oder größeres Volumen aufweisen als die Durchstechflasche.

Beim Entleeren der Durchstechflasche sinkt der Flüssigkeitsspiegel in der Durchstechflasche, bis ein Niveau erreicht ist, das unter der Öffnung (17) am Ende des ersten Leitungswegs (10) liegt. Ab diesem Zeitpunkt tritt Gas in den ersten Leitungsweg (10) ein. Ein Eintritt von Gas in den extrakorporalen Kreislauf soll vermieden werden. An den Zugabestellen (7, 8) vor der venösen Tropfkammer (6) ist die Vermeidung des Eintritts von Gas wünschenswert. Obwohl die Entfernung von Gasblasen zwar noch in der venösen Tropfkammer möglich ist, können Gasblasen im extrakorporalen Kreislauf trotzdem Komplikationen hervorrufen und führen im Laufe der Zeit auch zu einem unerwünschten Abfall des Flüssigkeitsspiegels in der venösen Tropfkammer. An der Zugabestelle (9) nach der venösen Tropfkammer ist die Vermeidung des Eintritts von Gas essentiell, weil keine Gasblasen in den Patienten infundiert werden dürfen. Daher empfiehlt es sich bei Nutzung dieser Zugabestelle noch einen Gasblasendetektor nach der Zugabestelle (9) im extrakorporalen Kreislauf bereit zu stellen.

Zur Vermeidung des Eintritts von Gas in den extrakorporalen Kreislauf wird in der bevorzugten Ausführungsform ist das Gassperrelement eine hydrophile Membran (12), die gasdicht in den ersten Leitungsweg (10) integriert ist. Eine hydrophile Membran ist in trockenem Zustand sowohl für Flüssigkeiten als auch für Gas passierbar. Nachdem eine hydrophile Membran von Flüssigkeiten, insbesondere wässrigen Flüssigkeiten, benetzt wurde, ist die hydrophile Membran im nassen Zustand für Gase im Wesentlichen undurchlässig. Hydrophile Membranen nehmen schnell Flüssigkeit auf und werden von ihnen durchnässt. Ein Maß für den Grad der Hydrophilie einer Membran bildet der CWST, dessen Messung in EP 313 348 beschrieben ist. Membranen mit einem CWST von größer als 72 dyn/cm gelten allgemein als hydrophil. Entsprechende Membranen können gewobene oder ungewobene poröse Strukturen sein. Die Membran kann aus natürlichem Material, wie z.B Baumwolle, Zellulose oder Hanf bestehen, besteht aber bevorzugt aus Polymeren hydrophiler Monomere, wie z.B. Vinylphosphonsäure, Vinylsulfonsäure, Acrylsäure und Methacrylsäure , Vinylpyridin, 3-Vinylpyridin, 2-Methyl-5-vinylpyridin, 3-Ethyl-4-vinylpyridin, 2, 3-Dimethyl-5-vinylpyridin, N-Vinylpyrrolidon, 2-Vinylpyrrolidon, N-Vinylpyrrolidin und 3-Vinylpyrrolidin, 2-Methacryloxyethylphosphorylcholin, Vinylalkohol, Alkylenoxide und Alkylenglykole. Hydrophile Monomere besitzen bevorzugt mindestens eine Löslichkeit von 50 g/l in Wasser.

Um in durchnässtem Zustand gasundurchlässig zu sein muss die Porengröße kleiner als 100 μm sein. Bevorzugt ist eine Porengröße von weniger als 15 μm und besonders bevorzugt ist eine Porengröße von weniger als 0,3 μm, weil in dieser Größenordnung zusätzlich das Passieren der Membran von Mikroorganismen verhindert wird. Die Gasundurchlässigkeit der genässten Membran wird durch den Blasendruck-Test (bubble point) nach folgender Formel bestimmt:

$$\Delta p = \frac{2\sigma \cdot \cos\theta}{r}$$

Δp = Druckdifferenz
σ = Oberflächenspannung der Flüssigkeit (Wasser=72,75mN/m)
θ = Benetzungswinkel des Materials
r = Porenradius

Um eine Dichtigkeit gegenüber Gas der genässten hydrophilen Membran zu gewährleisten, muss die die Membran so gewählt sein, dass die tatsächliche Druckdifferenz zwischen dem Druck im extrakorporalen Kreislauf und dem Druck im Gasreservoir ständig kleiner ist als Δp für die Membran nach oben stehender Formel berechnet wurde. Hierbei ist zu beachten, dass die Druckdifferenz bei extrakorporalen Kreisläufen nicht konstant ist, sondern beeinflusst durch die Pumpenbewegung schwankt.

Neben der Funktion als Gasbarriere funktioniert die hydrophile Membran (12) auch als ein den Fluss regulierendes Element. In vielen Fällen ist es gewünscht, dass das Medikament aus der Durchstechflasche über einen längeren Zeitraum appliziert wird. Die hydrophile Membran ist ein Filterelement und bildet einen Flusswiderstand, der den Fluss über einen gewissen Zeitraum regulieren kann. Der Fluss bemisst sich dabei als eine Funktion der Druckdifferenz, der Filterfläche und der Porengröße des Filterelements. Je nach Zugabestelle können bei einer durchschnittlichen Druckdifferenz die Filterfläche und die Porengröße des Filterelements so bemessen werden, dass die Applikation des Medikaments über einen vorbestimmten Zeitraum möglich ist.

In einer alternativen Ausführungsform ist das Gassperrelement ein Schwimmerventil (23). Geeignete Schwimmerventile sind dem Fachmann zum Beispiel aus der DE 28 30 845 bekannt. Bevorzugt besitzt ein Schwimmerventil ein zylindrisches Gehäuse, in dem eine auf wässrigen Flüssigkeiten schwimmende Kugel so angeordnet ist, dass sie während der Passage von Flüssigkeiten aufschwimmt und im flüssigkeitsfreien Zustand auf einem ringförmig im Gehäuse angeordneten Ventilsitz gasdicht aufsitzt.

Neben dem Gassperrelement (12, 23) können optional auch oberhalb und/oder unterhalb Gassperrelements weitere flussregulierende Elemente (21) angebracht sein. Solche Elemente, beispielsweise in Form von Ventilen oder Klemmen, können den Fluss auch vollständig stoppen. In bevorzugten Ausführungsformen besitzt die erfindungsgemäße medizinische Vorrichtung mindestens ein flussregulierendes Element unterhalb des Gassperrelemnts, wenn die Vorrichtung mit einer arteriellen Zugabestelle (7) verbunden ist. In einer anderen bevorzugten Ausführungsform besitzt die erfindungsgemäße medizinische Vorrichtung mindestens ein flussregulierendes Element oberhalb Gassperrelemtens, wenn die Vorrichtung mit einer venösen Zugabestelle (8, 9) verbunden ist.

Vorteilhafterweise kann in der direkten Nachbarschaft des Gassperrelements (12, 23) eine hydrophobe Membran (15) angeordnet sein, die nicht flüssigkeitsdicht im ersten Leitungsweg (10) angeordnet ist, sondern an der Seitenwand des ersten Leitungswegs eine Verbindung mit der umgebenden Luft bildet und somit zur Entlüftung des ersten Leitungswegs geeignet ist. Das Vorhandensein einer solchen hydrophoben Membran kann zum einen vorteilhaft zur Entlüftung des ersten Leitungswegs des Spikes beitragen direkt nach dem Anschluss der Durchstechflasche. Zum anderen besteht die Möglichkeit, dass sich im Inhalt der Durchstechflasche Mikroblasen gebildet haben, die die hydrophile Membran nicht passieren können, sich dort ansammeln und den Fluss durch die hydrophile Membran okkludieren. Die Anordnung der hydrophoben Membran in der Nachbarschaft der hydrophilen Membran (15) erlaubt die Entlüftung des an der hydrophilen Membran (12) angesammelten Gases. Wenn das Gassperrelement ein Schwimmerventil ist kann die hydrophobe Membran vorteilhafterweise auch im Gehäuse des Schwimmerventils angeordnet sein.

Die erfindungsgemäße medizinische Vorrichtung kann als integraler Bestandteil eines Hämodialyseschlauchsets ausgebildet sein. In einer bevorzugten Ausführungsform ist die medizinische Vorrichtung allerdings ein Adapter, der über eine Kupplungsstelle (20) an das Schauchset oder Kassettenset einer Hämodialysemaschine angeschlossen wird. Die Kupplung kann über eine Nadel oder eine Kanüle erfolgen, die durch ein Septum gestochen wird. Bevorzugt wird aber eine nadellose Kupplung, die das Verletzungsrisiko des Personals verringert. Nadellose Kupplungslösungen, beispielsweise über Luer-Konnektoren, sind aus dem Stand der Technik bekannt, wie beispielsweise aus der EP 1 673 135. Besonders bevorzugt sind Kupplungsmöglichkeiten, die die Sterilität der Zugabestelle gewährleisten.

Neben der arteriellen Zugabestelle (7) werden in der Dialysetechnik auch weitere Zugabestellen genutzt. Die arterielle Zugabestelle kann insbesondere nicht für Medikamente mit einem geringen Molekulargewicht genutzt werden, weil diese Substanzen durch den flussab der arteriellen Zugabestelle angeordneten Dialysator zumindest teilweise entfernt werden. Für die meisten niedermolekularen Medikamente bietet sich eine Zugabestelle (8) an, die zwischen dem Dialysator und der venösen Tropfkammer angeordnet ist. Diese Anordnung bietet eine zusätzliche Absicherung gegen eine Infusion von Gasblasen, die in der nachfolgenden Tropfkammer entfernt würden. Für einige Medikamente soll aber auch ein Kontakt mit der Umgebungsluft strikt vermieden werden, sei es um eine Oxidation zu vermeiden oder wegen der Toxizität des Medikaments. Für solche Medikamente bietet sich eine der venösen Tropfkammer nachgeordnete Zugabestelle (9) an.

Beide Zugabestellen im venösen Kreislauf des Schlauchsets sind nach der Blutfördereinrichtung (2) angeordnet. Im extrakorporalen Kreislauf herrschen nach der Blutfördereinrichtung, üblicherweise eine peristaltische Pumpe, andere Druckverhältnisse als vor der Fördereinrichtung. Im Gegensatz zur arteriellen Zugabestelle (7) herrscht an den beiden venösen Zugabestellen (8, 9) gegenüber der umgebenden Atmosphäre ein Überdruck im extrakorporalen Kreislauf. Um dennoch auch an den venösen Zugabestellen (8, 9) ein Einbringen des Medikaments in den Kreislauf zu gewährleisten, muss die Durchstechflasche mit einem Druck beaufschlagt werden, der den Druck im Inneren des extrakorporalen Kreislaufs an der Zugabestelle dauerhaft übersteigt. Hierzu wird ein Gasreservoir bereitgestellt in dem ein entsprechender Gasdruck herrscht.

Für das Bereitstellen eines Gasreservoirs mit einem bestimmten Druck werden verschiedene bevorzugte Ausführungsformen in Betracht gezogen: der Anschluss an eine externe Gasleitung mit im Wesentlichen konstantem pneumatischem Druck, die Beaufschlagung eines Gasbehälters mit Druck mittels einer mechanischen Pumpe, die Kompression eines verformbaren Gasbehälters, die Erzeugung von Gas in einem volumenbegrenzten Gasbehälter durch eine chemische Reaktion, wie beispielsweise aus der EP 1 673 135 bekannt.

In einer bevorzugten Ausführungsform besitzt das Gasreservoir, das mit Druck beaufschlagt wird, ein Überdruckventil, um zu vermeiden, dass der Druck einen kritischen Wert übersteigt. Insbesondere muss vermieden werden, dass die Druckdifferenz zum extrakorporalen Kreislauf den durch den Blasendruck der hydrophilen Membran vorgegebenen Wert übersteigt.

In einer weiteren bevorzugten Ausführungsform wird der Druck im Gasreservoir so gewählt, dass die Applikation des Inhalts der Durchstechflasche über einen bestimmten Zeitraum erfolgt, wobei die Erhöhung des Drucks den Zeitraum für die Applikation verringert.

In vielen modernen Hämodialysemaschinen existiert bereits eine Vorrichtung zum Einbringen eines pneumatischen Drucks in das Schlauchsystem. Üblicherweise wird der Druck in der venösen Tropfkammer (6) durch einen Druckmesser gemessen, der über eine Fluidverbindung mit dem Luftkissen im oberen Bereich der Tropfkammer verbunden ist. Häufig ist an dieser Fluidverbindung auch eine pneumatische Pumpe (P) angebracht, mit der durch zu- oder abpumpen von Luft der Flüssigkeitsspiegel in der Tropfkammer reguliert werden kann.

In einer besonders bevorzugten Ausführungsform wird eine in der Hämodialysemaschine bereits vorhandene Quelle für pneumatischen Druck verwendet, um einen Druck im Gasreservoir (14) zu erzeugen. Das Gasreservoir (14) ist in diesem Fall üblicherweise schlicht die Fluidverbindung zum zweiten Leitungsweg (11). Die Quelle für den pneumatischen Druck ist dann üblicherweise die Pumpe (P) zur Einbringung von Luft in die venöse Tropfkammer. Durch ein Ventil kann gesteuert werden, ob die Pumpe (P) mit dem Gasreservoir oder mit der venösen Tropfkammer in Verbindung steht.

In einer weiteren besonders bevorzugten Ausführungsform wird in der Fluidverbindung zwischen der Quelle für den pneumatischen Druck und dem zweiten Leitungsweg der Druck in dem so entstandenen Gasreservoir mittels einer weiteren Druckmessvorrichtung gemessen. Auf diese Weise kann durch Vergleich des Drucks im Gasreservoir und des Drucks in der venösen Tropfkammer die Druckdifferenz ermittelt werden. Daraufhin kann durch Einsatz der Pumpe der Druck im Gasreservoir so eingestellt werden, dass die Druckdifferenz unter Berücksichtung des Flusswiderstands in dem ersten Leitungsweg der medizinischen Vorrichtung genau so groß ist, um eine bestimmte Menge eines Medikaments pro Zeiteinheit zu verabreichen. Auf diese Weise ist die Verabreichung des Medikaments über einen Zeitraum in verbesserter Weise regulierbar. Außerdem kann so gewährleistet werden, dass die Druckdifferenz nicht den Blasendruck der hydrophilen Membran übersteigt.

Als eine weitere Ausführungsform ist unter Verwendung der Pumpe (P) denkbar, ein Ventil, das die Verbindung der Pumpe zum Gasreservoir oder der Tropfkammer steuert, so zu platzieren, dass eine ständige Fluidverbindung zwischen der Pumpe und einem Druckmesser besteht. Der eine Druckmesser kann dann wechselseitig, aber nicht gleichzeitig, den Druck in der Tropfkammer oder dem Gasreservoir erfassen. Vorteilhaft an dieser Ausführungsform ist die Einsparung eines Druckmessers.

In einer weiteren Ausführungsform besitzt die Hämodialysemaschine neben der bereits vohandnen Pumpe (P) eine weitere zusätzliche Pumpe, die den Druck im Gasreservoir steuert. Bevorzugt ist in einem solchen Fall, dass der Druck im Gasreservoir wiederum mittels eines Druckmessers gemessen wird und die Druckdifferenz zum Druck in der venösen Tropfkammer ermittelt wird. Auch diese Anordnung ermöglicht die Regulation der Druckdifferenz und somit die Verabreichung des Medikaments über einen bestimmten Zeitraum.

Im Folgenden werden Beispiele vorgestellt, die zur Erläuterung der Erfindung beitragen sollen, ohne die Erfindung in irgendeiner Weise einzuschränken.

Beispiel 1:

Der Blutfluss im extrakorporalen Kreislauf einer Hämodialysemaschine wird gestoppt durch Anhalten der Blutfördereinrichtung (2). Von der Aufnahmevorrichtung (16) einer mit der arteriellen Zugabestelle (7) versehenen erfindungsgemäßen medizinischen Vorrichtung wird die steril abdichtende Kappe entfernt. Eine Durchstechflasche enthaltend ein Medikament wird so auf die medizinische Vorrichtung aufgesteckt, dass der doppellumige Spike vollständig in die Durchstechflasche eindringt. Ein zuvor geschlossenes, flussregulierendes Element (21) in Form einer Klemme, die unterhalb der hydrophilen Membran angebracht ist, wird geöffnet. Es erfolgt eine Benetzung der hydrophilen Membran (12) mit Blut unter gleichzeitiger Entlüftung des ersten Leitungswegs (10) durch die hydrophobe Membran (15). Unmittelbar nach der Benetzung der hydrophilen Membran wird der Blutfluss durch Inbetriebnahme der Blutfördereinrichtung fortgesetzt. Bei einem Anschluss des zweiten Leitungswegs (11) der medizinischen Vorrichtung an ein Gasreservoir (14) mit atmosphärischem Druck, in diesem Falle Umgebungsluft, fällt der Druck an der arteriellen Zugabestelle (7) unter den atmosphärischen Druck ab. Durch die Druckdifferenz Δp wird der Inhalt der Durchstechflasche in den extrakorporealen Kreislauf überführt und das Volumen in der Durchstechflasche durch Umgebungsluft substituiert. Nachdem der gesamte Inhalt der Durchstechflasche in den extrakorporalen Kreislauf überführt wurde und auch das Volumen in dem ersten Leitungsweg (10) durch Luft substituiert wurde, stoppt der Fluidfluss durch die hydrophile Membran (12), die für Gas nicht passierbar ist. Das flussregulierende Element (21) wird verschlossen und die Aufnahmevorrichtung (16) durch eine steril abdichtende Kappe abgedeckt.

Beispiel 2:

Während des laufenden Blutflusses im extrakorporalen Kreislauf einer Hämodialysemaschine wird von der Aufnahmevorrichtung (16) einer mit einer venösen Zugabestelle (8, 9) verbundenen erfindungsgemäßen medizinischen Vorrichtung die steril abdichtende Kappe entfernt. Eine Durchstechflasche enthaltend ein Medikament wird so auf die medizinische Vorrichtung aufgesteckt, dass der doppellumige Spike vollständig in die Durchstechflasche eindringt.

Der zweite Leitungsweg (11) der medizinischen Vorrichtung wird mit einem Gasreservoir (14) verbunden, das einen pneumatischen Druck aufweist, der permanent höher ist als der Druck im extrakorporalen Kreislauf an der venösen Zugabestelle. Ein zuvor geschlossenes flussregulierendes Element (21) in Form einer Klemme, die oberhalb der hydrophilen Membran angebracht ist, wird geöffnet. Eine Benetzung der hydrophilen Membran (12) mit Blut unter gleichzeitiger Entlüftung des ersten Leitungswegs (10) durch die hydrophobe Membran (15) ist bereits zuvor durch die Verbindung mit dem extrakorporalen Blutkreislauf erfolgt, der an dieser Stelle einen Druck aufweist der größer als der atmosphärische Druck ist. Durch die Druckdifferenz $\Delta p$ zwischen dem Gasreservoir und dem extrakorporalen Kreislauf an der venösen Zugabestelle wird der Inhalt der Durchstechflasche in den extrakorporealen Kreislauf überführt und das Volumen in der Durchstechflasche durch Gas aus dem Gasreservoir substituiert. Nachdem der gesamte Inhalt der Durchstechflasche in den extrakorporalen Kreislauf überführt wurde und auch das Volumen in dem ersten Leitungsweg (10) durch Gas substituiert wurde, stoppt der Fluidfluss durch die hydrophile Membran (12), die für Gas nicht passierbar ist. Das flussregulierende Element (21) wird verschlossen und die Aufnahmevorrichtung (16) durch eine steril abdichtende Kappe abgedeckt

**Patentansprüche**

1. Medizinische Vorrichtung mit einer Durchstechvorrichtung zum Einführen in eine Durchstechflasche zur Medikamentenaufbewahrung mit einem ersten Leitungsweg (10) und einem zweiten Leitungsweg (11),
wobei der erste Leitungsweg (10) so ausgelegt ist, dass er an einem Ende in das innere Lumen der Durchstechflasche (22) mündet wenn die Durchstechvorrichtung vollständig in Durchstechflasche eindringt, und der erste Leitungsweg (10) mit dem anderen Ende an die Leitungswege eines extrakorporalen Blutkreislaufs einer Hämodialysemaschine (19) anschließt,
und wobei der zweite Leitungsweg (11) so ausgelegt ist, dass er an einem Ende in das Innere der Durchstechflasche mündet, wenn die Durchstechvorrichtung vollständig in Durchstechflasche eindringt, und der zweite Leitungsweg (11) mit dem anderen Ende mit einem Gasreservoir (14) so in Verbindung steht, dass eine Belüftung über den zweiten Leitungsweg stattfinden kann,
und wobei im ersten Leitungsweg ein Gassperrelement (12, 23) so angebracht ist, dass Flüssigkeiten das Gassperrelement passieren können, das Gassperrelement für Gase aber unpassierbar ist **dadurch gekennzeichnet, dass** der zweite Leitungsweg (11) an das Gasreservoir angeschlossen ist und der Druck im Gasreservoir den Druck an der Zugabestelle des extrakorporalen Kreislaufs übersteigt.

2. Medizinische Vorrichtung nach Anspruch 1, **dadurch gekennzeichnet, dass** die Vorrichtung integraler Bestandteil eines Schlauchsets oder eines Kassettensets für die Ausbildung eines extrakorporalen Blutkreislaufs einer Hämodialyse sind.

3. Medizinische Vorrichtung nach Anspruch 1, **dadurch gekennzeichnet, dass** die Vorrichtung ein Adapter zum Anschluss an das Schlauchset oder das Kassettenset eines extrakorporalen Kreislaufs einer Hämodialysemachine ist.

4. Medizinische Vorrichtung nach Anspruch 3, **dadurch gekennzeichnet, dass** der Adapter so ausgelegt ist, dass die Verbindung mit dem extrakorporalen Kreislauf nadellos erfolgt.

5. Medizinische Vorrichtung nach einem der vorhergehenden Ansprüche, **dadurch gekennzeichnet, dass** die medizinische Vorrichtung zusätzlich eine mit einer Kappe verschließbare Aufnahmevorrichtung (16) aufweist.

6. Medizinische Vorrichtung nach einem der vorhergehenden Ansprüche, **dadurch gekennzeichnet, dass** die medizinische Vorrichtung zusätzlich eine hydrophobe Membran (15) aufweist, die im ersten Leitungsweg so angeordnet ist, dass sie eine Verbindung zur Umgebungsluft bildet und dass sie den ersten Leitungsweg nicht flüssigkeitsdicht verschließt.

7. Medizinische Vorrichtung nach einem der vorhergehenden Ansprüche, **dadurch gekennzeichnet, dass** die medizinische Vorrichtung zusätzlich einen im zweiten Leitungsweg (11) angeordneten, bevorzugt hydrophoben, Sterilfilter (13) aufweist.

8. Medizinische Vorrichtung nach einem der vorhergehenden Ansprüche, **dadurch gekennzeichnet, dass** die medizinische Vorrichtung zusätzlich mindestens ein flussregulierendes Element (21), bevorzugt eine Klemme, aufweist, die im ersten Leitungsweg (10) oberhalb und/oder unterhalb des Gassperrelements (12, 23) angeordnet ist.

9. Medizinische Vorrichtung nach einem der vorhergehenden Ansprüche, **dadurch gekennzeichnet, dass** das Gasreservoir so ausgestaltet ist, dass der Gasdruck im Gasreservoir regulierbar ist.

10. Medizinische Vorrichtung nach einem der vorhergehenden Ansprüche, **dadurch gekennzeichnet, dass** das Gassperrelement eine hydrophile Membran ist, die im nassen Zustand der Membran für Gas nicht passierbar ist.

11. Medizinische Vorrichtung nach Anspruch 10, **da-**

**durch gekennzeichnet, dass** die hydrophile Membran eine Porengröße von kleiner als 100 μm aufweist, bevorzugt kleiner als 15 μm und besonders bevorzugt kleiner als 0,3 μm.

12. Medizinische Vorrichtung nach einem der Ansprüche 10 bis 11, **dadurch gekennzeichnet, dass** die hydrophile Membran aus einem hydrophilen polymeren Material gefertigt ist.

13. Medizinische Vorrichtung nach einem der Ansprüche 10 bis 12, **dadurch gekennzeichnet, dass** die hydrophile Membran gasdicht ist, bei einem Blasendruck, der der Druckdifferenz zwischen Gasreservoir und dem extrakorporalen Kreislauf entspricht.

14. Medizinische Vorrichtung nach einem der Ansprüche 1 bis 9, **dadurch gekennzeichnet, dass** das Gassperrelement ein Schwimmerventil ist.

15. Medizinische Vorrichtung nach Anspruche 15, **dadurch gekennzeichnet, dass** das Schwimmerventil ein zylindrisches Gehäuse besitzt, in dem eine auf wässrigen Flüssigkeiten schwimmende Kugel so angeordnet ist, dass sie während der Passage von Flüssigkeiten aufschwimmt und im flüssigkeitsfreien Zustand auf einem ringförmig im Gehäuse angeordneten Ventilsitz gasdicht aufsitzt.

## Claims

1. A medical apparatus having a puncture device for insertion into a vial for holding medications, having a first line path (10) and a second line path (11), wherein the first line path (10) is designed so that it opens at one end into the inside lumen of the vial (22) when the puncture device penetrates completely into the vial, the first line path (10) being connected at the other end to the line paths of an extracorporeal blood circulation of a hemodialysis machine (19), and wherein the second line path (11) is designed so that it opens at one end into the interior of the vial when the puncture device penetrates completely into the vial, and the second line path (11) is connected at the other end to a gas reservoir (14), so that ventilation can occur through the second line path, and wherein a gas barrier element (12, 23) is mounted in the first line path, so that liquids can pass through the gas barrier element but the gas barrier element is impassable for gases, **characterized in that** the second line path (11) is connected to the gas reservoir and the pressure in the gas reservoir exceeds the pressure at the dosing point of the extracorporeal circulation.

2. The medical apparatus according to Claim 1, **characterized in that** the apparatus is an integral component of a tube set or a cassette set for forming an extracorporeal blood circulation in hemodialysis.

3. The medical apparatus according to Claim 1, **characterized in that** the apparatus is an adapter for connection to the tube set or the cassette set of an extracorporeal circulation of a hemodialysis machine.

4. The medical apparatus according to Claim 3, **characterized in that** the adapter is designed so that the connection to the extracorporeal circulation is needle-free.

5. The medical apparatus according to any one of the preceding claims, **characterized in that** the medical apparatus additionally has a receptacle device (16) that can be closed with a cap.

6. The medical apparatus according to any one of the preceding claims, **characterized in that** the medical apparatus additionally has a hydrophobic membrane (15), which is disposed in the first line path, so that it forms a connection to the ambient air and does not seal the first line path in a fluid-tight manner.

7. The medical apparatus according to any one of the preceding claims, **characterized in that** the medical apparatus additionally has a sterile filter (13), which is preferably disposed in the second line path (11).

8. The medical apparatus according to any one of the preceding claims, **characterized in that** the medical apparatus additionally has at least one flow-regulating element (21), preferably a clamp, which is disposed above and/or below the gas barrier element (12, 23) in the first line path (10).

9. The medical apparatus according to any one of the preceding claims, **characterized in that** the gas reservoir is designed so that the gas pressure in the gas reservoir can be regulated.

10. The medical apparatus according to any one of the preceding claims, **characterized in that** the gas barrier element is a hydrophilic membrane through which no gas can pass when the membrane is wet.

11. The medical apparatus according to Claim 10, **characterized in that** the hydrophilic membrane has a pore size of less than 100 μm, preferably less than 15 μm, and especially preferably less than 0.3 μm.

12. The medical apparatus according to any one of Claims 10 to 11, **characterized in that** the hydrophilic membrane is manufactured from a hydrophilic polymeric material.

**13.** The medical apparatus according to any one of Claims 10 to 12, **characterized in that** the hydrophilic membrane is airtight at a bubble pressure corresponding to the pressure difference between the gas reservoir and the extracorporeal circulation.

**14.** The medical apparatus according to any one of Claims 1 to 9, **characterized in that** the gas barrier element is a float valve.

**15.** The medical apparatus according to Claim 15, **characterized in that** the float valve has a cylindrical housing in which balls floating on aqueous fluids are disposed, so that they float during the passage of liquids and sit with an airtight fit on a valve seat disposed in an annular form in the housing.

**Revendications**

**1.** Dispositif médical comprenant un dispositif de perçage destiné à être introduit dans un flacon à percer pour conserver des médicaments, comprenant un premier trajet d'introduction (10) et un second trajet d'introduction (11),
sachant que le premier trajet d'introduction (10) est ainsi conçu qu'il débouche à une extrémité dans le passage interne du flacon à percer (22) lorsque le dispositif de perçage pénètre complètement dans le flacon à percer, et le premier trajet d'introduction (10) se raccorde par l'autre extrémité aux trajets d'introduction d'un circuit sanguin extracorporel d'une machine de dialyse (19),
et sachant que le second trajet d'introduction (11) est ainsi conçu qu'il débouche à une extrémité à l'intérieur du flacon à percer lorsque le dispositif de perçage pénètre complètement dans le flacon à percer, et le second trajet d'introduction (11) est en liaison par l'autre extrémité avec un réservoir de gaz (14) de façon à ce qu'une ventilation puisse avoir lieu via le second trajet d'introduction,
et sachant qu'un élément d'arrêt de gaz (12, 23) est ainsi placé dans le premier trajet d'introduction (10) que des liquides puissent passer l'élément d'arrêt de gaz mais que les gaz ne puissent pas passer l'élément d'arrêt de gaz,
le second trajet d'introduction (11) est raccordé au réservoir de gaz et **caractérisé en ce que** la pression dans le réservoir de gaz dépasse la pression au point d'addition du circuit sanguin extracorporel.

**2.** Dispositif médical selon la revendication 1, **caractérisé en ce que** le dispositif fait partie intégrante d'un jeu de tuyaux ou d'un jeu de cassettes pour former un circuit sanguin extracorporel d'une hémodialyse.

**3.** Dispositif médical selon la revendication 1, **caractérisé en ce que** le dispositif est un adaptateur destiné à être raccordé au jeu de tuyaux ou au jeu de cassettes d'un circuit sanguin extracorporel d'une machine d'hémodialyse.

**4.** Dispositif médical selon la revendication 3, **caractérisé en ce que** l'adaptateur est ainsi conçu que la liaison a lieu sans aiguille avec le circuit sanguin extracorporel.

**5.** Dispositif médical selon l'une des revendications précédentes, **caractérisé en ce que** le dispositif médical présente en plus un dispositif de réception (16) pouvant se fermer avec un bouchon.

**6.** Dispositif médical selon l'une des revendications précédentes, **caractérisé en ce que** le dispositif médical présente en plus une membrane hydrophobe (15) qui est ainsi disposée dans le premier trajet d'introduction qu'elle forme une liaison vers l'air ambiant et qu'elle ne ferme pas le premier trajet d'introduction en étanchéité aux fluides.

**7.** Dispositif médical selon l'une des revendications précédentes, **caractérisé en ce que** le dispositif médical présente en plus un filtre stérile (13) de préférence hydrophobe disposé dans le second trajet d'introduction (11).

**8.** Dispositif médical selon l'une des revendications précédentes, **caractérisé en ce que** le dispositif médical présente en plus au moins un élément régulateur d'écoulement (21), de préférence une pince, qui est disposée dans le premier trajet d'introduction (10) au-dessus et/ou en-dessous de l'élément d'arrêt de gaz (12, 23).

**9.** Dispositif médical selon l'une des revendications précédentes, **caractérisé en ce que** le réservoir de gaz est ainsi conçu que la pression du gaz dans le réservoir de gaz peut être régulée.

**10.** Dispositif médical selon l'une des revendications précédentes, **caractérisé en ce que** l'élément d'arrêt de gaz est une membrane hydrophile qui ne laisse pas passer les gaz lorsque la membrane est mouillée.

**11.** Dispositif médical selon la revendication 10, **caractérisé en ce que** la membrane hydrophile présente une taille de pore inférieure à 100 $\mu$m, de préférence inférieure à 15 $\mu$m et particulièrement de préférence inférieure à 0,3 $\mu$m.

**12.** Dispositif médical selon l'une des revendications 10 à 11, **caractérisé en ce que** la membrane hydrophile est fabriquée dans un matériau polymère hydrophile.

**13.** Dispositif médical selon l'une des revendications 10 à 12, **caractérisé en ce que** la membrane hydrophile est étanche aux gaz avec une pression de soufflage qui correspond à la différence de pression entre le réservoir de gaz et le circuit sanguin extracorporel.

**14.** Dispositif médical selon l'une des revendications 1 à 9, **caractérisé en ce que** l'élément d'arrêt de gaz est un robinet flotteur.

**15.** Dispositif médical selon la revendication 15, **caractérisé en ce que** le robinet flotteur possède un boîtier cylindrique dans lequel une bille qui flotte sur les liquides aqueux est ainsi disposée qu'elle flotte pendant le passage de liquides et dans l'état sans liquides, elle repose de façon étanche aux gaz sur un siège de soupape annulaire disposé dans le boîtier.

**Fig. 1**

**Fig. 2**

**Fig. 3**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 3837298
- US 5330425 A
- EP 966631 A
- US 4500309 A
- EP 532432 A
- WO 8707159 A
- US 20080097315 A1
- IT T02009000455 A
- EP 313348 A
- DE 2830845
- EP 1673135 A